(19) Europäisches Patentamt
European Patent Office
Office européen des brevets

(11) Veröffentlichungsnummer: **0 007 089**
**B1**

(12) # EUROPÄISCHE PATENTSCHRIFT

(45) Veröffentlichungstag der Patentschrift: **16.09.81**

(51) Int. Cl.³: **C 07 C 103/58,** A 01 N 37/22

(21) Anmeldenummer: **79102359.1**

(22) Anmeldetag: **10.07.79**

(54) **Acylanilide mit herbicider und fungicider Wirkung, Verfahren zu ihrer Herstellung und ihre Verwendung.**

(30) Priorität: **11.07.78 DE 2830351**

(43) Veröffentlichungstag der Anmeldung: **23.01.80 Patentblatt 80/2**

(45) Bekanntmachung des Hinweises auf die Patenterteilung: **16.09.81 Patentblatt 81/37**

(84) Benannte Vertragsstaaten: **AT BE CH DE FR GB IT NL SE**

(56) Entgegenhaltungen:
**DE-A 1 116 469**
**FR-A-1 554 984**
**US-A-3 210 420**
**US-A-3 843 793**

**CHEMICAL ABSTRACTS, Vol. 81, Nr. 25, 23. Dezember 1974, Columbus, Ohio, USA, ROGER F. C. BROWN et al.: «Methylene ketenes and methylenecarbenes», Seite 520, Zusammenfassung Nr. 169248v**

(73) Patentinhaber: **Consortium für elektrochemische Industrie GmbH, Zielstattstrasse 20, D-8000 München 70 (DE)**

(72) Erfinder: **Häberle, Norman, Dr. Dipl.-Chem., Willibaldstrasse 51a, D-8000 München 21 (DE)**
Erfinder: **Kinzel, Peter, Jägerkampstrasse 21a, D-8152 Westerham/Feldkirchen (DE)**
Erfinder: **Wick, Manfred, Dr. Dipl.-Chem., Mesnergasse 5, D-8162 Schliersee (DE)**

(56) Entgegenhaltungen:
**HELVETICA CHIMICA ACT. Vo. 57, Fase 8, 1974, P. SCHIESS et al.: «Reversible, thermische Isomerisierung zwischen α,β,γ,δ-ungesättigten Aldehyden und Alkenylketenen durch (1,5)-H-Verschiebung, Valenhisomerisierung von cis-Dienonen, V (1)», Seiten 2583-2597**

**TETRAHEDRON LETTERS, Nr. 46, 1975, J. DE BOER et al.: «ω-Carbinollactanes in regioselective olefin-synthesis», Seiten 4039-4042**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 089 B1

ACTORUM AG.

# Acylanilide mit herbicider und fungicider Wirkung, Verfahren zu ihrer Herstellung und ihre Verwendung

Acylanilide der allgemeinen Formel

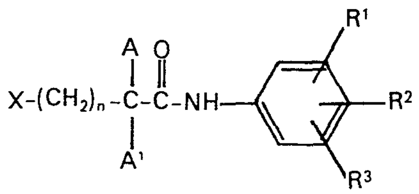

I

worin bedeuten:

X einen Äthenyl- oder Äthinylrest, wobei im Äthenylrest ein oder zwei Wasserstoffatome und im Äthinylrest ein Wasserstoffatom durch Methyl und/oder Äthyl substituiert sein können;

A und $A^1$ gleiche oder verschiedene Reste aus der Gruppe der Wasserstoff-, Methyl- und Äthylreste;

n 0 oder 1;

$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest;

$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen,

können wegen ihrer ausgeprägten herbiciden und/oder fungiciden Wirkung vorteilhaft als Pflanzenschutzmittel eingesetzt werden. Einige Verbindungen sind zudem neu.

Die Acylanilide leiten sich von Acyl- oder Carbonsäureresten der allgemeinen Formel

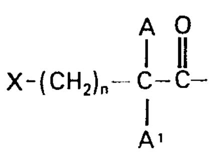

II

ab, wobei X, A, $A^1$ und n die vorstehend angegebenen Bedeutungen besitzen.

Durch die Formeln I und II werden alle möglichen cis-trans-Isomeren umfasst, die Ansprüche beziehen sich demgemäss auf einzelne Isomeren oder Isomerengemische.

Die Klasse der Acylanilide ist für ihre herbicide und/oder fungicide Wirksamkeit allgemein bekannt. Eine Reihe solcher Wirkstoffe werden im Pflanzenschutz bereits vielfältig eingesetzt. Beispielsweise wird in R. Wegler, Chemie der Pflanzenschutz- und Schädlingsbekämpfungsmittel, Berlin, Heidelberg, New York, 1970 bzw. 1976, Band 2, Seite 311 ff, eine Anzahl solcher Verbindungen genannt, insbesondere einige solche, die einen substituierten Anilinrest sowie einen in zwei-Stellung substituierten Acylrest besitzen. Bisher sind aber nur Acylanilide gesättigter oder alpha-beta-ungesättigter Carbonsäuren beschrieben worden, das heisst, letztere sind dadurch charakterisiert, dass die Mehrfachbindung des Acylrestes in Konjugation zur Carboxylgruppe steht:

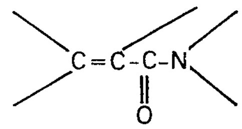

Die DE-B 11 16 469 beansprucht beispielsweise Methacrylanilide, die DE-A 22 49 547 z.B. anilinmodifizierte Anilide gesättigter und alpha-beta-ungesättigter Carbonsäuren.

Die FR-A-1554984 betrifft Acylanilide mit herbiciden Wirkungen, die sich von Alkyl-, Alkenyl- und Alkinylcarbonsäuren ableiten. Jedoch handelt es sich bei den genannten Verbindungen lediglich um Äquivalente der vorstehend abgehandelten Substanzen (vgl. auch Tabelle 2 dieser Anmeldung), in denen die gegebenenfalls vorhandenen Mehrfachbindungen stets in Konjugation mit der Carbonylgruppe stehen.

Die US-A-3 843 793 beschreibt Mittel zur Behandlung von nichtbösartiger prostatischer Hypertrophie und zur chemischen Kastration männlicher Tiere, die als Wirkstoff Anilide von Alkenylcarbonsäuren mit nicht in Konjugation zur Carbonylgruppe stehender Doppelbindung enthalten. Diese Mittel können auch zur Kontrolle von tierischen Schädlingen eingesetzt werden, da deren Fortpflanzung herabgesetzt oder verhindert wird, die vorhandene Population wird jedoch nicht verkleinert.

Es wurde nun gefunden, dass überraschenderweise Acylanilide der allgemeinen Formel I, in denen die Mehrfachbindung des Carbonsäureesters nicht in Konjugation zur Amidgruppe -CO-N= steht, wesentlich höhere Selektivität beim Einsatz als Pflanzenschutzmittel zeigen. Überdies sind bei der Anwendung der erfindungsgemässen Acylanilide wegen weit höherer Wirksamkeit (siehe Beispiele 5 und 6, insbesondere Wirkstoff 1 – Vergleichssubstanz D) Einsparungen bezüglich der Aufwandmengen möglich, wodurch die Probleme in bezug auf Pflanzenschutzmittelrückstände, Metaboliten und Abbauzeiten stark vermindert werden.

Besonders wirksam als Pflanzenschutzmittel zeigen sich die Verbindungen, abgeleitet von Acylresten der allgemeinen Formel II, bei denen

X einen $Y_2C{=}CH$-Rest mit gleichen oder verschiedenen Resten Y mit der Bedeutung H, $CH_3$ oder $C_2H_5$ bzw. einen $H\text{-}C{\equiv}C$-Rest,

A und $A^1$ Wasserstoff- und/oder Methylreste,

n 0 oder 1

bedeuten. Diese Verbindungen bzw. ihre Verwendungen als Pflanzenschutzmittel sind deshalb bevorzugt. Weiterhin bevorzugt sind bzw. werden eingesetzt solche Acylanilide, in deren Anilinrest

$R^1$ ein H-, F-, Cl- oder Br-Atom, einen Methyl-, Äthyl-, Propyl- oder iso-Propyl-, einen Trifluormethyl-, einen Methoxy-, einen Phenoxy- oder chlorsubstituierten Phenoxy- oder einen Nitrorest,
$R^2$ und $R^3$ H- und/oder Cl-Atome und/oder Methyl- und/oder Äthylreste
bedeuten.

Aus dieser Gruppe sind solche Acylanilide der allgemeinen Formel I bevorzugt, worin X, A, $A^1$, n, $R^1$, $R^2$ und $R^3$ die oben angegebenen Bedeutungen haben und bei denen der Anilinrest nicht ein- oder zweifach substituiert ist. In diesen Fällen sind die 4'-, 2',6'-, 3',4'- und 3',5'-Substitutionen bevorzugt. Besonders bevorzugt sind die 3',4'-substituierten, insbesondere die 3',4'-Dichlor-Anilidverbindungen.

Besonders bevorzugt werden ausserdem Anilide von Acylresten der vorstehend genannten allgemeinen Formel II eingesetzt, worin A, $A^1$, n und Y wie vorstehend angegeben definiert sind, X die Bedeutung $Y_2C{=}CH-$ besitzt und die Anzahl der Kohlenstoffatome in den Resten Y zusammen kleiner oder gleich zwei ist.

Beispiele für bevorzugte Verbindungen sind in Tabelle 1 aufgeführt. Insbesondere seien genannt Anilide der 2,2-Dimethylpent-3-ensäure, vor allem 2,2-Dimethylpent-3-ensäure-3',4'-dichloranilid.

Als Herbicide besonders wirksame Acylanilide, deren Einsatz auch besonders bevorzugt ist, sind solche der allgemeinen Formel I, worin X, A, $A^1$, $R^1$, $R^2$ und $R^3$ die oben angegebenen Bedeutungen haben, mindestens A oder $A^1$ einen Methylrest, insbesondere A und $A^1$ je einen Methylrest bedeuten und n 0 ist.

Die Verbindungen besitzen neben ihrer herbiciden auch eine graduell unterschiedliche fungicide Wirksamkeit. Diese fungicide Wirksamkeit tritt besonders hervor, wenn n 1 bedeutet, da dann die herbicide Wirkung oft abgeschwächt ist.

Die erfindungsgemässen bzw. erfindungsgemäss verwendeten Acylanilide lassen sich herstellen beispielsweise durch Anilidierung der entsprechenden Carbonsäurechloride, gegebenenfalls in Gegenwart säurebindender Mittel, wie tertiärer Amine, von Pyridinen, Alkali- und Erdalkalicarbonaten, -hydroxyden oder -oxyden, insbesondere Soda, Pottasche, Magnesia, gebranntem, gelöschtem oder kohlensaurem Kalk oder Tiräthylamin.

Andere Methoden der Herstellung sind zum Beispiel die Umsetzung geeigneter entsprechender Grignardverbindungen mit gegebenenfalls substituierten Phenylisocyanaten, die Anilidierung von Estern durch direkte Umsetzung mit Hilfe von Alkoholaten, insbesondere der Alkalimetalle, z.B. Natrium- oder Kaliummethanolat, -äthanolat, -propanolat, oder die Anilidierung der freien Säuren unter katalytischer Wasserabspaltung und Auskreisen des Wassers mittels azeotroper Destillation.

Die Carbonsäuren bzw. ihre Ester, die zum Teil bereits bekannt sind, können hergestellt werden beispielsweise durch Malonestersynthesen, Grignardsynthesen, Oxydation entsprechender Aldehyde oder Ketone, die ihrerseits z.B. durch Aldol-Kondensationen herstellbar sind, oder durch Reformatzkysynthesen und dann gegebenenfalls nach bekannten Methoden zu den Säurechloriden umgesetzt werden.

Einzelne Herstellungsmethoden werden in den Beispielen angegeben. Weitere Vorschriften finden sich in Lehrbüchern der präparativen organischen Chemie.

Die erfindungsgemässen bzw. erfindungsgemäss verwendeten Acylanilide, weiterhin auch Wirkstoffe genannt, können als herbicide und/oder fungicide Mittel oder als Saatgut-Beizmittel eingesetzt werden.

Die hier beschriebenen Wirkstoffe wirken sowohl über die Wurzeln als auch über die Blätter der Pflanzen, wobei manchmal eine der genannten Wirkungen grösser ist als die andere.

Weiterhin können die hier beschriebenen Verbindungen als solche einzeln oder im Gemisch ausgebracht werden. Im allgemeinen werden sie jedoch als Mischungen mit festen oder flüssigen Verdünnungsmitteln oder als Lösungen in festen oder flüssigen Lösungsmitteln verwendet mit Wirkstoffgehalten von 0,01 bis 95 Gew.-%.

Die Mischungen bzw. Lösungen werden im allgemeinen als Emulsionskonzentrate, Pasten, Spritzpulver, Stäubemittel, Granulate oder Microkapseln hergestellt. Die Ausbringung kann in jeder geeigneten Form erfolgen, beispielhaft seien genannt: Giessen, Verspritzen, Versprühen, Verstreuen, Verstäuben, Bestreichen, Behandeln des Saatgutes (Beizen). Die Emulsionskonzentrate, Pasten und Spritzpulver werden üblicherweise mit Wasser verdünnt, um Spritzbrühen zu erhalten.

Emulsionskonzentrate und Pasten enthalten im allgemeinen 10–60 Gew.-%, vorzugsweise 15–40 Gew.-%, Wirkstoff, 2–25 Gew.-% Dispergierhilfsstoffe und organische Lösungsmittel und/oder Wasser; Spritzpulver enthalten meistens 10–80 Gew.-%, vorzugsweise 15–70 Gew.-%, Wirkstoff, 1–10 Gew.-% Dispergierhiflsstoffe und 10–89 Gew.-% inerte Bestandteile; Stäubemittel, Granulate und Mikrokapseln enthalten neben inerten Bestandteilen, Bindemitteln und/oder Überzugsstoffen 1–10 Gew.-%, vorzugsweise 5–10 Gew.-%, Wirkstoff.

Die Konzentrationen der Bestandteile in den erwähnten Formulierungen addieren sich jeweils zu 100 Gew.-%.

Beispielhaft genannt seien als erfindungsgemäss anwendbare Dispergierhilfsstoffe: Alkyl- und Arylsulfonate, Methylcellulose, polymere Sulfonsäuren und deren Salze, Polyalkohole, Fettsäureester, Fettalkoholäther, Fettamine; als organische Lösungsmittel: Alkohole, wie Äthanol, Butanole, Dimethylformamid (DMF), Dimethylsulfoxid (DMSO), N-Methylpyrrolidon, Aromaten, wie Toluol und Xylole;
als inerte Bestandteile: Kaolin, China-Clay, Talkum, Calciumcarbonat, hochdisperse Kieselsäure, Kieselgele, Kieselgur, Diatomeenerde, Bims, Ziegelsplitt, Maisschrot, Verdickungsmittel, wie Stärke und Carboxymethylcellulose;

als Bindemittel: Magnesiumsulfat, Gips, Gummiarabikum.

Beispielsweise werden hier beschriebene Wirkstoffe zur Verwendung als Herbicide wie folgt formuliert:

1) Emulsionskonzentrat:
20 Gew.-% Wirkstoff (Wirkstoff 10)
20 Gew.-% handelsübliches äthoxyliertes Anhydrosorbitmonolaurat («Tween Twenty», eingetragenes Warenzeichen)
60 Gew.-% DMF

2) Spritzpulver:
20 Gew.-% Wirkstoff (Wirkstoff 8)
5 Gew.-% Ammoniumligninsulfonat («Totanin», eingetragenes Warenzeichen)
10 Gew.-% Natriumoleylmethyltaurid («Arkopon Tkonz», eingetragenes Warenzeichen)
65 Gew.-% Kaolin

Die hier beschriebenen Wirkstoffe bzw. Formulierungen könen allein oder in Kombination mit anderen Pesticiden, mit Schädlingsbekämpfungsmitteln und mit Pflanzennährstoffen ausgebracht werden.

Im allgemeinen werden die Wirkstoffe in Mengen von 0,5–5 kg/ha, vorzugsweise 0,5–4 kg/ha, ausgebracht.

Die Herbicidwirkung der hier beschriebenen, besonders wirksamen Acylanilide lassen vor allem in Baumwoll-, Mais-, Reis-, Wintergetreide-, Ackerbohnen-, Karotten- und anderen Gemüsekulturen selektive Unkrautbekämpfung zu.

Die Wirksamkeit der hier beschriebenen Acylanilide ist so gross, dass bereits wesentlich geringere Aufwandmengen als bisher gegen Unkräuter und -gräser voll wirksam sind.

Die hier beschriebenen Mittel, die Acylanilide der allgemeinen Formel I enthalten, wobei X, Y, A, $A^1$, $R^1$, $R^2$,$R^3$ und n die vorstehend definierten Bedeutungen zukommen, sind herbicid wirksam gegen mono- und dikotyle Pflanzen, wie z.B. gegen Samenunkräuter und Gräser. Als einzelne Beispiele dafür seien genannt: Gänsefussarten, Kornblume, Franzosenkraut, Ackerstiefmütterchen, Kreuzkraut, Hederich, Ehrenpreis, Ackersenf, Kamille, Vogelmiere, Flughafer, Ackerfuchsschwanz, einjährige Rispe, Hühnerhirse, Bluthirse, Saatwucherblume, Amaranth, Taubnessel, Knöterich, Klettenlabkraut.

Die Herbicidwirkstoffe können im Vorsaat-, im Vorauflauf- und im Nachauflaufverfahren angewendet werden.

Die fungiciden Wirkstoffe wirken als Beizmittel und als Kontaktmittel.

Ihre Wirksamkeit bei einmaliger Anwendung wurde im Vergleich zu drei bekannten Handelspräparaten (A–C) und einem zu Wirkstoff 1 strukturisomeren Anilid einer alpha-beta-ungesättigten Carbonsäure (D) in Labor-, Gewächshaus- und Freilandversuchen getestet (Tabelle 2). Die Handelspräparate A–C werden seit längerer Zeit zur Bekämpfung von Unkräutern im Nachauflaufverfahren eingesetzt.

Tabelle 1

| Wirkstoff-nummer | Verbindungsname | Summenformel | Schmelzbereich bzw. Schmelzpunkt in °C |
|---|---|---|---|
| 1 | 2-Methylbut-3-ensäure-3′,4′-dichloranilid | $C_{11}H_{11}Cl_2NO$ | 83-85 |
| 2 | 2-Methylbut-3-ensäure-3′-chlor-4′-fluoranilid | $C_{11}H_{11}ClFNO$ | 65-67 |
| 3 | 2-Methylbut-3-ensäure-3′-trifluormethyl-4′-chloranilid | $C_{12}H_{11}ClF_3NO$ | 66-68 |
| 4 | 2,2-Dimethylbut-3-ensäure-3′,4′-dichloranilid | $C_{12}H_{13}Cl_2NO$ | 61-62 |
| 5 | 2,2-Dimethylbut-3-insäure-3′,4′-dichloranilid | $C_{12}H_{11}Cl_2NO$ | 95 |
| 6 | 2-Methylpent-3-ensäure-3′,4′-dichloranilid | $C_{12}H_{13}Cl_2NO$ | 35-37 |
| 7 | 2,2-Dimethylpent-3-ensäure-2′,6′-diäthylanilid | $C_{17}H_{25}NO$ | 158 |
| 8 | 2,2-Dimethylpent-3-ensäure-3′,4′-dichloranilid | $C_{13}H_{15}Cl_2NO$ | 75-76 |
| 9 | 2,2-Dimethylpent-3-ensäure-3′-chlor-4′-isopropylanilid | $C_{16}H_{22}ClNO$ | 69-72 |
| 10 | 2,2-Dimethylpent-3-ensäure-3′-chlor-4′-fluoranilid | $C_{13}H_{15}ClFNO$ | 51-52 |
| 11 | 2,2-Dimethylpent-3-ensäure-3′-chlor-4′-bromanilid | $C_{13}H_{15}BrClNO$ | 55-56 |
| 12 | 2,2-Dimethylpent-3-ensäure-3′,5′-dichloranilid | $C_{13}H_{15}Cl_2NO$ | 56-59 |
| 13 | 2,2-Dimethylpent-3-ensäure-3′-chlor-4′-methoxyanilid | $C_{14}H_{18}ClNO_2$ | 68-70 |
| 14 | 2,2,4-Trimethylpent-3-ensäure-3′,4′-dichloranilid | $C_{14}H_{17}Cl_2NO$ | 99-101 |
| 15 | 2,2-Dimethylhex-3-ensäure-3′,4′-dichloranilid | $C_{14}H_{17}Cl_2NO$ | 64-69 |
| 16 | 2-Methylbut-3-ensäure-4′(p-Chlorphenoxy)anilid | $C_{17}H_{16}ClNO_2$ | 95 |
| 17 | But-3-ensäure-3′,4′-dichloranilid | $C_{10}H_9Cl_2NO$ | 40 |
| 18 | 2-Methylpent-4-ensäure-3′,4′-dichloranilid | $C_{12}H_{13}Cl_2NO$ | 82 |
| 19 | 2-Methylpent-4-ensäure-3′-chlor-4′-methylanilid | $C_{13}H_{16}ClNO$ | 70 |
| 20 | 2,2-Dimethylpent-4-ensäure-3′,4′-dichloranilid | $C_{13}H_{15}Cl_2NO$ | 75 |
| 21 | 2-Methylbut-3-ensäure-4′-nitroanilid | $C_{11}H_{12}N_2O_3$ | 135 |
| 22 | 2-Methylpent-4-insäure-3′,4′-dichloranilid | $C_{12}H_{11}Cl_2NO$ | 87 |
| 23 | 2-Methylbut-3-ensäure-3′,5′-dichloranilid | $C_{11}H_{11}Cl_2NO$ | 40-50 |
| 24 | 2,2-Dimethylpent-3-ensäureanilid | $C_{13}H_{17}NO$ | 49 |

Tabelle 1 (Fortsetzung)

| Wirkstoff-nummer | Verbindungsname | Summenformel | Schmelzbereich bzw.Schmelz-punkt in °C |
|---|---|---|---|
| 25 | 2,2-Dimethylpent-3-ensäure-3'-chlor-4'-methylanilid | $C_{14}H_{18}ClNO$ | 70–72 |
| 26 | 2,2-Dimethylpent-3-ensäure-3',5'-dichlor-4'-methoxyanilid | $C_{14}H_{17}Cl_2NO_2$ | 79–81 |
| 27 | 2,2,3-Trimethylpent-3-ensäure-3',4'-dichloranilid | $C_{14}H_{17}Cl_2NO$ | 68–69 |

Tabelle 2

| Vergleichs-substanz | |
|---|---|
| A | 2-Methylvaleriansäure-3'-chlor-4'-methylanilid aus DE-B-11 60 236 («Pentanochlor»*, «Solan»*) |
| B | Methacrylsäure-3,4-dichloranilid aus DE-B-11 16 469 bzw. GB-A-8 85 043 («Chloranocryl«*) |
| C | 2,2-Dimethylvaleriansäure-4'-chloranilid aus DE-C-11 66 547 («Potablan»*, «Monalide»*) |
| D | 2-Methylbut-2-ensäure-3',4'-dichloranilid (cis-Isomeres) = Tiglinsäure-3',4'-dichloranilid |

* eingetragene Warenzeichen

Die folgenden Beispiele sollen die Herstellung und die Anwendung der hier beschriebenen Acylanilide anhand einzelner Verbindungen erläutern.

Mengen- und Konzentrationsangaben beziehen sich auf das Gewicht, soweit nichts anderes angegeben ist.

Beispiel 1
Darstellung von 2-Methylbut-3-ensäure-3',4'-dichloranilid (Wirkstoff 1)

Die Lösung einer Grignardverbindung aus 54,5 g (0,6 Mol) käuflichem 3-Chlorbuten-1 und 14,6 g (0,6 Mol) Magnesium in 500 ml absolutem Äther wird mit gemahlenem Trockenreis versetzt, das Gemisch nach erfolgter Umsetzung vorsichtig hydrolysiert und schwach angesäuert. Durch Phasentrennung, alkalisches Ausschütteln der Ätherphase, Phasentrennung, erneutes Ansäuern und nochmaliges Ausäthern wird die rohe Carbonsäure gewonnen. Sie wird durch Destillation bei 53,3 Pa (0,4 Torr) und 70 °C gereinigt. Die Ausbeute beträgt 45% d.Th.

Die Säure wird mit Thionylchlorid in 80% Ausbeute in das entsprechende Säurechlorid umgewandelt.

Das Säurechlorid wird in absolutem Toluol gelöst und diese Lösung zu einem äquimolaren Gemisch aus 3,4-Dichloranilin und Triäthylamin in toluolischer Lösung bei 0–5 °C zugetropft. Nach beendeter Zugabe wird eine Stunde auf 50–60 °C erwärmt, vom ausgeschiedenen Triäthylammoniumhydrochlorid abfiltriert und vom Filtrat das Lösungsmittel abdestilliert. Der Rückstand wird im Ölpumpenvakuum bei 1,33 Pa (0,01 Torr) und 157 °C destilliert. Das gesuchte Anilid wird dabei mit 72,6% Ausbeute erhalten. Es kristallisiert nach kurzer Zeit und weist danach einen Schmelzbereich von 83–85 °C auf.

Beispiel 2
Darstellung von 2,2-Dimethylbut-3-ensäureaniliden

Nach einer Vorschrift von L. Bouveault (Bull. soc. chim. France [3], 21, 1964) wird in einer Reformatzkysynthese 2-Brom-2-methylpropionsäureäthylester mit Acetaldehyd umgesetzt. Aus dem erhaltenen 2,2-Dimethyl-3-hydroxybuttersäureester kann nach der gleichen Vorschrift mit Hilfe von $P_2O_5$ in inertem Lösungsmittel oder mit Pyridin/$POCl_3$ Wasser abgespalten werden. Dadurch wird 2,2-Dimethylbut-3-ensäureäthylester erhalten. Diese Verbindung kann in üblicher Weise (z.B. wie in Beispiel 1) nach der Verseifung des Esters mit Alkalilauge mit Hilfe von Thionylchlorid in das entsprechende Säurechlorid umgewandelt werden und dieses dann mit dem gewünschten Anilinderivat zum gesuchten Acylanilid umgesetzt werden. Im Falle des 3,4-Dichloranilids (Verbindung 4) beträgt die Ausbeute bei der Verseifung des Esters 83,2% d.Th., bei der Säurechlorierung 73,5% d.Th. un bei der Anilidierung 96,3% d.Th.

Beispiel 3
Darstellung von 2-Methylpent-4-ensäureaniliden

Aus 348,4 g käuflichem 2-Methylmalonsäurediäthylester und 149 g Propargylchlorid wird mit Hilfe von Natrium in absolutem Alkohol eine Malonestersynthese nach Beilstein, 4. Auflage, Bd. 2, Hauptwerk, S. 485, durchgeführt, die in 83% Ausbeute 2-Methyl-2-propargyl-malonsäurediäthylester ergibt.

Nach üblicher alkalischer Verseifung und Decarboxylierung der freien Dicarbonsäure wird mit 74,4% Gesamtausbeute die freie 2-Methylpent-4-insäure (Kochpunkt bei 1,8 kPa [14 Torr]: 106–109 °C) erhalten, die wie z.B. in Beispiel 1 beschrieben in eines der gewünschten Anilide überführt werden kann.

Beispiel 4
Darstellung von 2,2-Dimethylbut-3-insäureaniliden

An 2,2-Dimethylbut-3-ensäureäthylester (vgl. Beispiel 2) wird in Chloroform Brom angelagert. Dabei erhält man in 52% Ausbeute 2,2-Dimethyl-3,4-di-brombuttersäure (Kochpunkt bei 1,6 kPa

[12 Torr]: 119–124°C), aus der mit methanolischer KOH Bromwasserstoff abgespalten wird, so dass 2,2-Dimethyl-3-brombut-3-en-säure (60% Ausbeute) entsteht. In eine Suspension von 21,4 g Natriumamid in absolutem DMSO (Dimethylsulfoxid) werden unter Rühren 27 g der zuletzt genannten Säure zugetropft, wobei heftige Reaktion stattfindet. Man rührt noch 5 Stunden bei 50°C und dann über Nacht bei Zimmertemperatur. Danach wird der Inhalt des Reaktionskolbens in Eiswasser gegossen und ausgeäthert. Anschliessend wird mit HCl angesäuert und erneut ausgeäthert, die Ätherphase getrocknet und destilliert. Man erhält dabei in 32% Ausbeute etwa 5 g 2,2-Dimethylbut-3-insäure (Kochpunkt bei 1,6 kPA [12 Torr]: 95–98°C), die wie in Beispiel 1 weiterverarbeitet werden kann.

Die Anwendungsversuche wurden wie folgt durchgeführt:

Die Beispiele 5 und 6 zeigen die gegenüber den Vergleichsstoffen weit überlegene Wirksamkeit der hier beschriebenen Wirkstoffe im Nachauflaufverfahren, insbesondere den Einfluss der Lage der Doppelbindung im Acylrest bei sonst gleichem Molekülbau.

Die Wirkstoffe wurden als Emulsionskonzentrat bzw. Spritzpulver formuliert und nach dem Verdünnen der Formulierung mit Wasser auf junge Pflanzen gespritzt, die die im folgenden beschriebenen Wachstumsstadien erreicht hatten.

Bei zweikeimblättrigen (dikotylen) Pflanzen war neben den primären Keimblättern das erste echte Blattpaar entwickelt, bei einkeimblättrigen (monokotylen) Pflanzen waren mindestens zwei Blätter ausgebildet.

Die behandelten Pflanzen wurden 14 Tage nach der einmaligen Spritzung mit den Wirkstoffen abschliessend auf Schädigung bzw. Abtötung bonitiert.

Aus Beispiel 6 ist zudem ersichtlich, dass die für die Unkrautvernichtung notwendigen Wirkstoffmengen der hier beschriebenen Acylanilide von Kulturpflanzen der Gruppe I toleriert werden.

Beispiel 5

Schädigung bzw. Abtötung von Pflanzen in % durch Wirkstoffe bzw. Vergleichsmittel bei Anwendung von 2 kg/ha Aktivsubstanz im Nachauflaufverfahren, Freilandversuche, Wirkstoffe als verdünnte Emulsionskonzentrate eingesetzt

| | Sommergerste | Flughafer | Sorghumhirse | Klettenlabkraut |
|---|---|---|---|---|
| Wirkstoff 1 | 70 | 98 | 70 | 70 |
| Wirkstoff 4 | 70 | 95 | 70 | 85 |
| Wirkstoff 6 | 70 | 85 | 70 | 95 |
| Wirkstoff 8 | 70 | 98 | 85 | 95 |
| Wirkstoff 9 | 85 | 85 | 85 | 98 |
| Wirkstoff 10 | 95 | 100 | 85 | 98 |
| Vergleich A | 25 | 45 | 10 | 45 |
| Vergleich B | 0 | 0 | 0 | 30 |
| Vergleich D | 25 | 40 | 20 | 25 |

Beispiel 6

Schädigung bzw. Abtötung verschiedener Pflanzengruppen in % durch Wirsktoffe und Vergleichsmittel bei Anwendung von 0,5 kg/ha Aktivsubstanz (Nachauflaufverfahren), Gewächshausversuche, Wirkstoffe als suspendiertes Spritzpulver eingesetzt

| | I Kulturpflanzen | | | II widerstandsfähige dikotyle Pflanzen | | | | | | III monokotyle Pflanzen | | | |
|---|---|---|---|---|---|---|---|---|---|---|---|---|---|
| | Weizen | Mais | Karotten | Amaranth | Klettenlabkraut | Knöterich | Kornblume | Saatwucherblume | Taubnessel | Ackerfuchsschwanz | einj. Rispe | Hühnerhirse | Bluthirse |
| Wirkst. 1 | 0 | 0 | 0 | 98 | 50 | 85 | 100 | – | 100 | – | 40 | 98 | 90 |
| Wirkst. 6 | 0 | 0 | 10 | 98 | 60 | 100 | – | – | 95 | 55 | 60 | 95 | 75 |
| Wirkst. 8 | 0 | 0 | 10 | 100 | 95 | 100 | 70 | 55 | 95 | 70 | 75 | 98 | 100 |
| Wirkst. 9 | 0 | 10 | – | 98 | 70 | 100 | 100 | 100 | 100 | 45 | 70 | 90 | 98 |
| Wirkst. 10 | 0 | 10 | – | 85 | 60 | 100 | 60 | 20 | 98 | – | 55 | 90 | 80 |
| Vergl. A | 0 | 10 | 0 | 40 | 40 | – | – | 0 | 70 | 40 | 0 | 80 | 65 |
| Vergl. B | 0 | 10 | 20 | – | 0 | 75 | 0 | – | 95 | 0 | 10 | 55 | – |
| Vergl. C | 0 | 0 | 0 | 30 | – | 70 | 40 | 0 | 50 | 10 | 20 | 20 | 20 |

In Beispiel 7 wird die erhöhte Wirksamkeit der hier beschriebenen Wirkstoffe gegenüber den Handelspräparaten im Vorauflaufverfahren verdeutlicht.

Hierzu wurden die Wirkstoffe bereits vor dem Aufkeimen der im Boden befindlichen Samenkörner auf die Bodenfläche ausgebracht. Die Samenkörner der Kulturpflanzen und Unkräuter wurden dazu zunächst in mineralischem, humusarmem Ackerboden ausgesät und leicht mit Bodenpartikeln abgedeckt. Unmittelbar nach der Aussaat wurden die hier beschriebenen Wirkstoffe in Form von Spritzpulvern in Wasser suspendiert und auf die vegetationsfreie Bodenoberfläche gleichmässig aufgespritzt. Vier Wochen nach der Behandlung wurden die Pflanzen abschliessend auf Schädigung bzw. Abtötung bonitiert, wobei inzwischen unbehandelt herangewachsene Kontrollpflanzen als Bezug dienten.

Beispiel 7

Wirksamkeit in % bei Anwendung von 2 kg/ha Aktivsubstanz im Vorauflaufverfahren

| | Vogelmiere | Kamille | Taubnessel | Flughafer | einjährige Rispe | Senf |
|---|---|---|---|---|---|---|
| Wirkstoff 8 | 100 | 100 | 100 | 98 | 98 | 100 |
| Wirkstoff 9 | 95 | 98 | 98 | 95 | 98 | 95 |
| Wirkstoff 10 | 85 | 75 | 80 | 85 | 90 | 95 |
| Vergleich A | 0 | 0 | 0 | 0 | 0 | 0 |
| Vergleich B | 0 | 30 | 10 | 0 | 20 | 40 |

Die Beispiele 8 und 9 dienten zur Ermittlung fungicider Eigenschaften der hier beschriebenen Acylanilide.

Beispiel 8

Suspensionen der Wirkstoffe wurden mit einer Mikropipette auf Objektträger mit Hohlschliffen gegeben und getrocknet. Dann wurden auf die gleichmässigen Wirkstoffbeläge gleiche Volumina von Sporensuspensionen der Testpilze (Schneeschimmel = fusarium nivale) aufgetropft, so dass Wirkstoffkonzentrationen von 500 ppm erreicht wurden. Das Sporenmaterial war von gleichaltrigen Agar-Kulturen geerntet worden.

Die Bebrütung erfolgte in geschlossenen Feuchtschalen bei Raumtemperatur (ca. 20 °C) über einen Zeitraum von 24–48 Stunden. Bei der mikroskopischen Auswertung wurde das Verhältnis der gekeimten und der nichtgekeimten Sporen ermittelt.

| | Abtötungsrate in % |
|---|---|
| Wirkstoff 17 | 100 |
| Wirkstoff 18 | 100 |
| Wirkstoff 19 | 100 |
| Wirkstoff 21 | 100 |
| Wirkstoff 23 | 100 |
| Vergleich B | 0 |
| Vergleich C | 0 |

Beispiel 9

Künstlich mit Sporen von Weizensteinbrand (Tilletia tritici) infizierte Weizenkörner werden mit einer Talkum-Wirkstoff-Mischung (1:1) so gemischt, dass eine gleichmässige Verteilung von 0,2% Wirkstoff entsteht. 24 Stunden nach der Behandlung wird 2 Tage im Dunkeln bei 14–17 °C bebrütet. Dann werden die Sporen durch Abdrükken in lehmiger Erde von den Körnern getrennt und nochmals bei 14–17 °C 5–8 Tage bebrütet. Anschliessend wird unter dem Mikroskop die Zahl der gekeimten Sporen bestimmt. Die gefundene Zahl ist das Mass für die Wirksamkeit des Mittels (0 Sporen 100%, 400 und mehr Sporen 0% Wirksamkeit).

| | Wirksamkeit in % |
|---|---|
| Wirkstoff 18 | 100 |
| Wirkstoff 20 | 100 |
| Wirkstoff 22 | 100 |
| Wirkstoff 23 | 100 |
| Vergleich A | 60 |
| Vergleich C | 40 |

**Patentansprüche** für die Vertragsstaaten: BE, CH, DE, FR, GB, IT, NL, SE

1. Verwendung eines oder mehrerer Acylanilide(s) der allgemeinen Formel

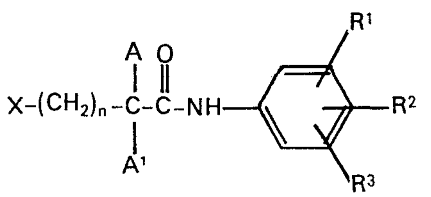

I

worin bedeuten:

X einen Äthenyl- oder Äthinylrest, wobei im Äthenylrest ein oder zwei Wasserstoffatome und im Äthinylrest ein Wasserstoffatom durch Methyl und/oder Äthyl substituiert sein können,

A und $A^1$ gleiche oder verschiedene Reste aus der Gruppe der Wasserstoff-, Methyl- und Äthylreste,

n 0 oder 1,

$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,

$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen,
als Pflanzenschutzmittel.

2. Ausführungsform nach Anspruch 1, dadurch gekennzeichnet, dass Acylanilid(e) verwendet wird (werden), in dessen (deren) Acylrest der allgemeinen Formel

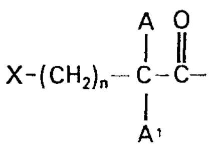
II

X einen $Y_2C{=}CH$-Rest mit gleichen oder verschiedenen Resten Y mit der Bedeutung H, $CH_3$ oder $C_2H_5$,
n 0 oder 1
und
A und $A^1$ Wasserstoff- und/oder Methylreste bedeuten.

3. Ausführungsform nach Anspruch 2, dadurch gekennzeichnet, dass die Zahl der Kohlenstoffatome in oben genannten Resten Y zusammengezählt kleiner oder gleich 2 ist.

4. Verwendung durch eines oder mehrerer Acylanilide(s) der allgemeinen Formel

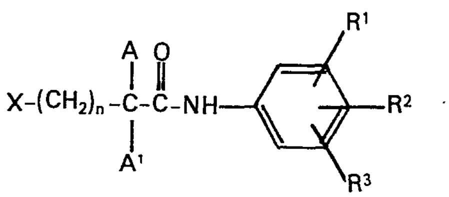
I

worin bedeuten:
X einen Äthenyl- oder Äthinylrest, wobei im Äthenylrest ein oder zwei Wasserstoffatome und im Äthinylrest ein Wasserstoffatom durch Methyl und/oder Äthyl substituiert sein können,
A oder $A^1$ einen Methyl-, insbesondere aber A und $A^1$ je einen Methylrest,
n 0,
$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen
als herbizides Mittel.

5. Verwendung eines oder mehrerer Acyanilide(s) der allgemeinen Formel

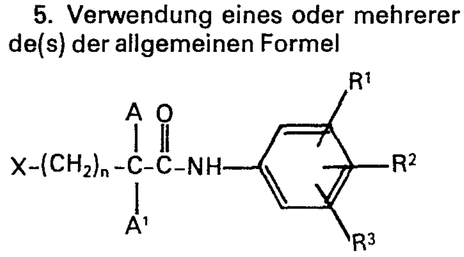
I

worin bedeuten:
X einen Äthenyl- oder Äthinylrest, wobei im Äthenylrest ein oder zwei Wasserstoffatome und im Äthinylrest ein Wasserstoffatom durch Methyl und/oder Äthyl substituiert sein können,
A und $A^1$ gleiche oder verschiedene Reste aus der Gruppe der Wasserstoff-, Methyl- und Äthylreste,
n 1,
$R^1$ ein Wasserstoff-, ein Halogenatom, ein gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyrest mit 1-4 C-Atomen,
als fungicides Mittel.

6. Ausführungsform nach einem der Ansprüche 1 bis 5, dadurch gekennzeichnet, dass im Anilinrest der darin verwendeten Acylanilide
$R^1$ ein H-, F-, Cl- oder Br-Atom, einen Methyl-, Äthyl-, Propyl- oder iso-Propyl-, einen Trifluormethyl-, einen Methoxy-, einen Phenoxy- oder chlorsubstituierten Phenoxyrest oder einen Nitrorest,
$R^2$ und $R^3$ H- und/oder Cl-Atome und/oder Methyl- und/oder Äthylreste
bedeuten.

7. Pflanzenschutzmittel enthaltend 2,2-Dimethylpent-3-ensäure-3',4'-dichloranilid.

8. Acylanilide der allgemeinen Formel

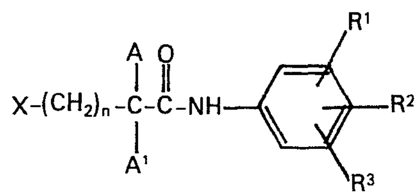
I

worin bedeuten:
X einen $H{-}C{\equiv}C$-Rest, dessen Wasserstoffatom durch Methyl oder Äthyl ersetzt sein kann,
A und $A^1$ gleiche oder verschiedene Reste aus der Gruppe der Wasserstoff-, Methyl- und Äthylreste,
n 0 oder 1,
$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen,

9. Pflanzenschutzmittel gekennzeichnet durch einen Gehalt an einem oder mehreren Acylanilid(en) gemäss Anspruch 8.

10. Verfahren zur Herstellung der Verbindungen nach Anspruch 8 durch Umsetzung der freien Carbonsäuren

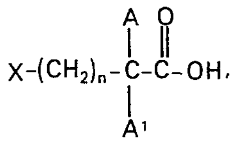

worin X, A, A1 und n die vorstehend definierten Bedeutungen haben, zu entsprechenden Carbonsäurechloriden und anschliessende Anilidierung, gegebenenfalls in Gegenwart säurebindender Mittel.

**Patentansprüche** für den Vertragsstaat: AT

1. Verwendung eines oder mehrerer Acylanilide(s) der allgemeinen Formel

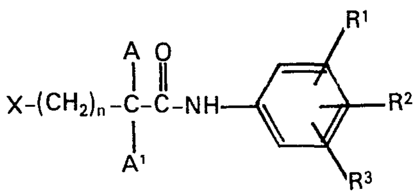

I

worin bedeuten:
X einen Äthenyl- oder Äthinylrest, wobei im Äthenylrest ein oder zwei Wasserstoffatome und im Äthinylrest ein Wassrstoffatom durch Methyl und/oder Äthyl substituiert sein können,
A und $A^1$ gleiche oder verschiedene Reste aus der Grupe der Wasserstoff-, Methyl- und Äthylreste,
n 0 oder 1,
$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen,
als Pflanzenschutzmittel.

2. Ausführungsform nach Anspruch 1, dadurch gekennzeichnet, dass Acylanilide(e) verwendet wird (werden), in dessen (deren) Acylrest der allgemeinen Formel

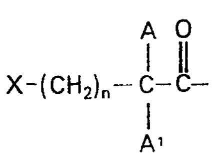

II

X einen $Y_2C{=}CH$-Rest mit gleichen oder verschiedenen Resten Y mit der Bedeutung H, $CH_3$ oder $C_2H_5$,
n 0 oder 1
und
A und $A^1$ Wasserstoff- und/oder Methylreste bedeuten.

3. Ausführungsform nach Anspruch 2, dadurch gekennzeichnet, dass die Zahl der Kohlenstoffatome in oben genannten Resten Y zusammengezählt kleiner oder gleich 2 ist.

4. Verwendung durch eines oder mehrerer Acylanilide(s) der allgemeinen Formel

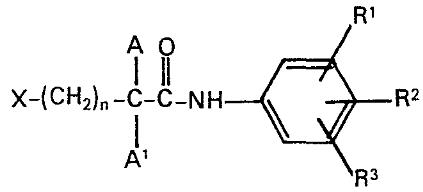

I

worin bedeuten:
X einen Äthenyl- oder Äthinylrest, wobei im Äthenylrest ein oder zwei Wasserstoffatome und im Äthinylrest ein Wasserstoffatom durch Methyl und/oder Äthyl substituiert sein können,
A oder $A^1$ einen Methyl-, insbesondere aber A und $A^1$ je einen Methylrest,
n 0,
$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen
als herbizides Mittel.

5. Verwendung eines oder mehrerer Acyanilide(s) der allgemeinen Formel

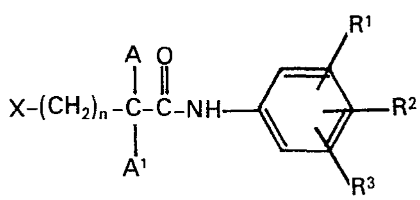

I

worin bedeuten:
X einen Äthenyl- oder Äthinylrest, wobei im Äthenylrest ein oder zwei Wasserstoffatome und im Äthinylrest ein Wasserstoffatom durch Methyl und/oder Äthyl substituiert sein können,
A und $A^1$ gleiche oder verschiedene Reste aus der Gruppe der Wasserstoff-, Methyl- und Äthylreste,
n 1,
$R^1$ ein Wasserstoff-, ein Halogenatom, ein gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyrest mit 1-4 C-Atomen und Alkoxyrest mit 1-4 C-Atomen,
als fungicides Mittel.

6. Ausführungsform nach einem der Ansprüche 1 bis 5, dadurch gekennzeichnet, dass im Anilinrest der darin verwendeten Acylanilide
$R^1$ ein H-, F-, Cl- oder Br-Atom, einen Methyl-,

Äthyl-, Propyl- oder iso-Propyl-, einen Trifluormethyl-, einen Methoxy-, einen Phenoxy- oder chlorsubstituierten Phenoxyrest oder einen Nitrorest,
$R^2$ und $R^3$ H- und/oder Cl-Atome und/oder Methyl- und/oder Äthylreste
bedeuten.

7. Pflanzenschutzmittel enthaltend 2,2-Dimethylpent-3-ensäure-3′,4′-dichloranilid.

8. Verfahren zur Herstellung von Acylaniliden der allgemeinen Formel

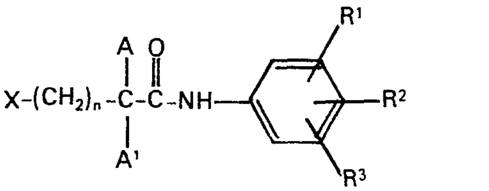

I

worin bedeuten:
X einen H-C≡C-Rest, dessen Wasserstoffatom durch Methyl oder Äthyl ersetzt sein kann,
A und $A^1$ gleiche oder verschiedene Reste aus der Gruppe der Wasserstoff-, Methyl- und Äthylreste,
n 0 oder 1,
$R^1$ ein Wasserstoff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen,
durch Umsetzung der freien Carbonsäuren

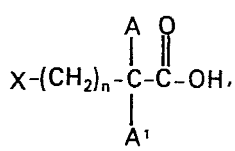

worin X, A, $A^1$ und n die vorstehend definierten Bedeutungen haben, zu entsprechenden Carbonsäurechloriden und anschliessende Anilidierung, gegebenenfalls in Gegenwart säurebindender Mittel.

9. Pflanzenschutzmittel gekennzeichnet durch einen Gehalt an einem oder mehreren Acylaniliden der allgemeinen Formel

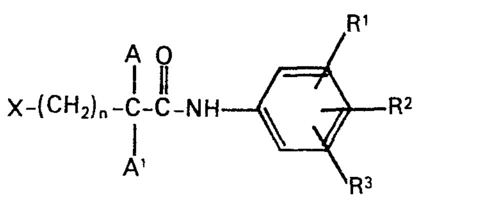

I

worin bedeuten:
X einen Äthinylrest, wobei das Wasserstoffatom durch Methyl oder Äthyl substituiert sein kann,
A und $A^1$ gleiche oder verschiedene Reste aus der Gruppe der Wasserstoff-, Methyl- und Äthylreste,
n 0 oder 1,
$R^1$ ein Wasserstroff-, ein Halogenatom, einen gerad- oder verzweigtkettigen, gegebenenfalls halogensubstituierten Alkylrest mit 1-4 C-Atomen, einen Alkoxyrest mit 1-4 C-Atomen, einen gegebenenfalls halogensubstituierten Oxyarylrest oder einen Nitrorest,
$R^2$ und $R^3$ gleiche oder verschiedene Reste aus der Gruppe der Wasserstoff-, Halogen-, Alkylreste mit 1-4 C-Atomen und Alkoxyreste mit 1-4 C-Atomen.

10. Pflanzenschutzmittel nach Anspruch 9, dadurch gekennzeichnet, dass in Formel I
X einen H-C≡C-Rest,
n 0 oder 1,
A und $A^1$ Wasserstoff- und/oder Methylreste,
$R^1$ ein H-, F-, Cl- oder Br-Atom, einen Methyl-, Äthyl-, Propyl- oder iso-Propyl-, einen Trifluormethyl-, einen Methoxy-, einen Phenoxy- oder chlorsubstituierten Phenoxyrest oder einen Nitrorest,
$R^2$ und $R^3$ H- und/oder Cl-Atome und/oder Methyl- und/oder Äthylreste
bedeuten.

**Claims** for the Contracting states: BE, CH, DE, FR, GB, IT, NL, SE

1. Use of one or several acylanilides of the following general formula as a plant protective agent,

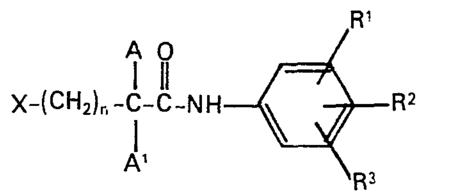

I

in which formula
X represents an ethenyl radical or an ethynyl radical, wherein in the ethenyl radical one or two hydrogen atoms, and in the ethynyl radical one hydrogen atom, can be substituted by methyl and/or ethyl,
A and $A^1$ respresent the same or different radicals selected from the group consisting of hydrogen radicals, methyl radicals and ethyl radicals,
n is 0 or 1,
$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical, or a nitro radical, and
$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

2. Mode of execution according to claim 1, characterized in that acylanilides are used, the

acyl radical of which corresponds to the general formula

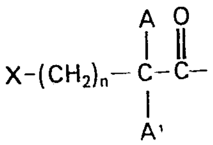

II

and wherein

X represents a $Y_2C{=}CH$ radical having the same of different radicals Y which denote H, $CH_3$ or $C_2H_5$,

n is 0 or 1, and

A and $A^1$ represent hydrogen radicals and/or methyl radicals.

3. Mode of execution according to claim 2, characterized in that the number of carbon atoms in the above-mentioned radicals Y together is less than, or equal to, 2.

4. Use of one or several acylanilides of the following general formula as a herbicidal agent

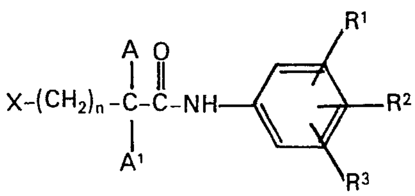

I

in which formula

X respresents an ethenyl radical or an ethynyl radical, wherein in the ethenyl radical one or two hydrogen atoms, and in the ethynyl radical one hydrogen atom, can be substituted by methyl and/or ethyl,

A or $A^1$ represent a methyl radical, but especially A and $A^1$ each represent a methyl radical,

n is 0,

$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical or a nitro radical, and

$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

5. Use of one or several acylanilides of the following general formula as a fungicidal agent

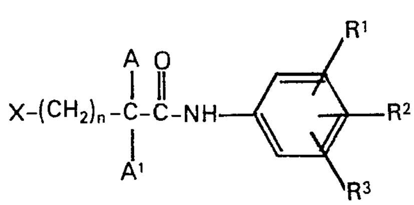

I

in which formula

X represents an ethenyl radical or an ethynyl radical, wherein in the ethenyl radical one or two hydrogen atoms, and in the ethynyl radical one hydrogen atom, can be substituted by methyl and/or ethyl,

A and $A^1$ represent the same or different radicals selected from the group consisting of hydrogen radicals, methyl radicals and ethyl radicals,

n is 1,

$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halgoen-substituted oxyaryl radical, or a nitro radical, and

$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

6. Mode of execution according to one or more of claims 1 to 5, characterized in that in the aniline radical of the acylanilides used in said claims

$R^1$ represents a H-atom, a F-atom, a Cl-atom or a Br-atom, a methyl radical, an ethyl radical, a propyl radical or an iso-propyl radical, a trifluoromethyl radical, a methoxy radical, a phenoxy radical or a chloro-substituted phenoxy radical, or a nitro radical, and

$R^2$ and $R^3$ represent H and/or Cl atoms and/or methyl radicals and/or ethyl radicals.

7. Plant protective agent containing 2,2-Dimethylpent-3-enoic acid-3′, 4′-dichloroanalide.

8. Acylanilides of the general formula

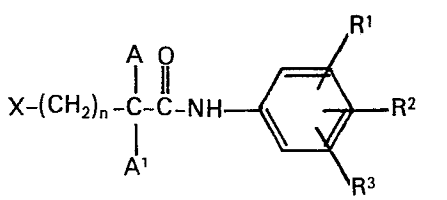

I

in which

X represents a H-C≡C- radical, the hydrogen atom of which an be substituted by methyl or ethyl,

A and $A^1$ represent the same or different radicals selected from the group consisting of hydrogen radicals, methyl radicals and ethyl radicals,

n is 0 or 1,

$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical, or a nitro radical, and

$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

9. Plant protective agents, characterized by a content of one or several of the acylanilides according to claim 8.

10. Process for the manufacture of the compounds according to claim 8 by reacting the free carboxylic acids

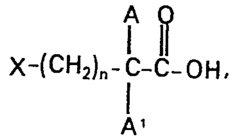

in which X, A, A[1] and n have the meanings defined above, to form corresponding carboxylic acid chlorides and by subsequent anilidation, optionally in the presence of acid-binding agents.

**Claims** for the Contracting state: AT

1. Use of one or several acylanilides of the following general formula as a plant protective agent,

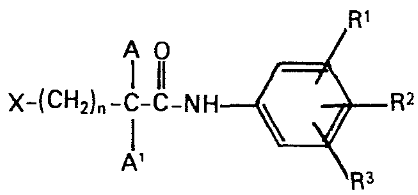
I

in which formula
X represents an ethenyl radical or an ethynyl radical, wherein in the ethenyl radical one or two hydrogen atoms, and in the ethynyl radical one hydrogen atom, can be substituted by methyl and/or ethyl,
A and $A^1$ respresent the same or different radicals selected from the group consisting of hydrogen radicals, methyl radicals and ethyl radicals,
n is 0 or 1,
$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical, or a nitro radical, and
$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

2. Mode of execution according to claim 1, charcterized in that acylanilides are used, the acyl radical of which corresponds to the general formula

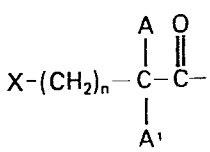
II

and wherein
X represents a $Y_2C{=}CH$ radical having the same or different radicals Y which denote H, $CH_3$ or $C_2H_5$,
n is 0 or 1, and
A and $A^1$ represent hydrogen radicals and/or methyl radicals.

3. Mode of execution according to claim 2, characterized in that the number of carbon atoms in the above-mentioned radicals Y together is less than, or equal to, 2.

4. Use of one or several acylanilides of the following general formula as a herbicidal agent

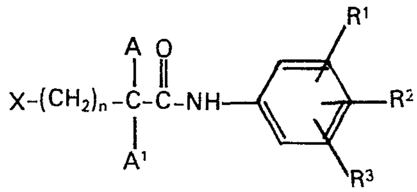
I

in which formula
X represents an ethenyl radical or an ethynyl radical, wherein in the ethenyl radical one or two hydrogen atoms, and in the ethynyl radical one hydrogen atom, can be substituted by methyl and/or ethyl,
A or $A^1$ represent a methyl radical, but especially A and $A^1$ each represent a methyl radical,
n is 0,
$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical or a nitro radical, and
$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

5. Use of one or several acylanilides of the following general formula as a fungicidal agent

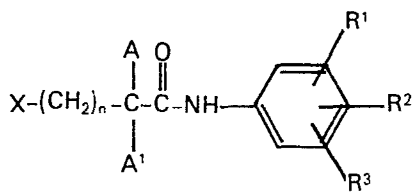
I

in which formula
X represents an ethenyl radical or an ethynyl radical, wherein in the ethenyl radical one or two hydrogen atoms, and in the ethynyl radical one hydrogen atom, can be substituted by methyl and/or ethyl,
A and $A^1$ represent the same or different radicals selected from the group consisting of hydrogen radicals, methyl radicals and ethyl radicals,
n is 1,
$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical, or a nitro radical, and
$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

6. Mode of execution according to one or more of claims 1 to 5, characterized in that in the aniline radical of the acylanilides used in said claims
$R^1$ represents a H-atom, a F-atom, a Cl-atom or a Br-atom, a methyl radical, an ethyl radical, a propyl radical or an iso-propyl radical, a trifluoromethyl radical, a methoxy radical, a phenoxy radical or a chloro-substituted phenoxy radical, or a nitro radical, and
$R^2$ and $R^3$ represent H and/or Cl atoms and/or methyl radicals and/or ethyl radicals.

7. Plant protective agent containing 2,2-Dimethylpent-3-enoic acid-3',4'-dichloroanilide.

8. Process for the manufacture of acylanilides of the general formula

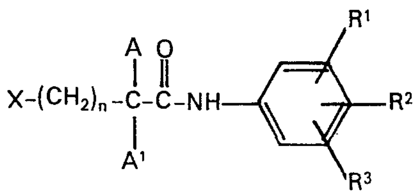
I

in which
X represents a H-C≡C-radical, the hydrogen atom of which can be substituted by methyl or ethyl,
A and $A^1$ represent the same or different radicals selected from the group consisting of hydrogen radicals, methyl radicals and ethyl radicals,
n is 0 or 1,
$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical, or a nitro radical, and
$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms
by reacting the free carboxylic acids

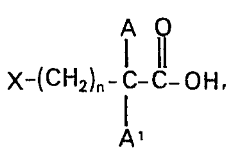

in which X, A, $A^1$ and n have the meanings defined above, to form corresponding carboxylic acid chlorides and by subsequent anilidation, optionally in the presence of acid-binding agents.

9. Plant protective agents, characterized by a content of one or several acylanilides of the general formula

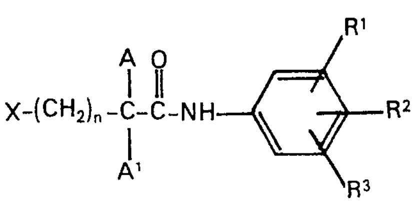
I

in which
X represents an ethynyl radical wherein the hydrogen atom can be substituted by methyl or ethyl,
A and $A^1$ represent the same or different radicals selected from the group consisting of hydrogen radicals, methyl radicals or ethyl radicals,
n is 0 or 1,
$R^1$ represents a hydrogen atom, a halogen atom, a straight-chain or branched-chain, optionally halogen-substituted, alkyl radical having from 1 to 4 carbon atoms, an alkoxy radical having from 1 to 4 carbon atoms, an optionally halogen-substituted oxyaryl radical, or a nitro radical, and
$R^2$ and $R^3$ represent the same or different radicals selected from the group consisting of hydrogen radicals, halogen radicals, alkyl radicals having from 1 to 4 carbon atoms and alkoxy radicals having from 1 to 4 carbon atoms.

10. Plant protective agents according to claim 9, characterized in that in formula I
X represents a H-C≡C-radical,
n is 0 or 1,
A and $A^1$ represent hydrogen radicals and/or methyl radicals,
$R^1$ represents a H-atom, a F-atom, a Cl-atom, or a Br-atom, amethyl radical, an ethyl radical, a propyl radical or an iso-propyl radical, a trifluoromethyl radical, a methoxy radical, a phenoxy radical or a chloro-substituted phenoxy radical, or a nitro radical, and
$R^2$ and $R^3$ represent H and/or Cl atoms and/or methyl radicals and/or ethyl radicals.

**Revendications** pour les Etats contractants: BE, CH, DE, FR, GB, IT, NL, SE

1. Utilisation comme agents phytosanitaires d'un ou de plusieurs acylanilides de formule générale

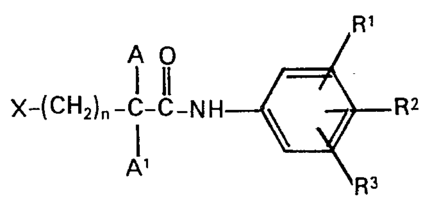
I

dans laquelle
X représente un radical éthényle ou éthynyle, un ou deux atomes d'hydrogène du radical éthényle ou un atome d'hydrogène du radical éthynyle pouvant être remplacés par un méthyle et/ou un éthyle;
A et $A^1$, qui peuvent être identiques ou différents l'un de l'autre, représentent chacun l'hydrogène ou le radical méthyle ou éthyle;
n est le nombre 0 ou 1;
$R^1$ désigne un atome d'hydrogène ou d'halogène, un alkyle en $C_{1-4}$ à chaîne doite ou ramifiée, éventuellement halogéné, un alcoxy en $C_{1-4}$, un radical aryloxy éventuellement halogéné ou un groupe nitro; et
$R^2$ et $R^3$, qui peuvent être identiques ou différents

l'un de l'autre, représentent chacun l'hydrogène ou un halogène ou un alkyle ou un alcoxy en $C_{1-4}$.

2. Variante de la revendication 1, caractérisée en ce que l'on utilise des acylanilides dans le radical acyle desquels, de formule générale

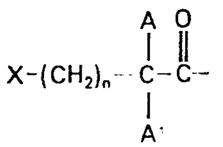

II

X est un radical $Y_2C{=}CH-$ à restes Y identiques ou différentes, à savoir H, $CH_3$ ou $C_2H_5$,
n est le nombre 0 ou 1
et
A et $A^1$ représentent chacun l'hydrogène ou un méthyle.

3. Variante de la revendication 2, caractérisée en ce que le nombrte des atomes de carbone de l'ensemble des deux restes Y est égal ou inférieur 2.

4. Utilisation comme herbicides d'un ou de plusieurs acylanilides de formule générale

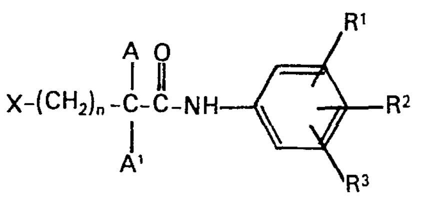

I

dans laquelle
X représente un radical éthényle ou éthynyle, un ou deux atomes d'hydrogène du radical éthényle ou un atome d'hydrogène du radical éthynyle pouvant être remplacés par un méthyle et/ou un éthyle,
A ou $A^1$, mais de préférence les deux, représentent chacun un méthyle,
n = 0,
$R^1$ est un atome d'hydrogène ou d'halogène, un radical alkyle à chaîne droite ou ramifiée en $C_{1-4}$, éventuellement halogéné, un alcoxy en $C_{1-4}$, un aryloxy éventuellement hylogéné ou un groupe nitro, et
$R^2$ et $R^3$, qui peuvent être identiques ou différents l'un de l'autre, sont choisis parmi l'hydrogène, les halogènes et des radicaux alkyles et alcoxy en $C_{1-4}$.

5. Utilisation comme fongicides d'un ou de plusieurs acylanilides de formule générale

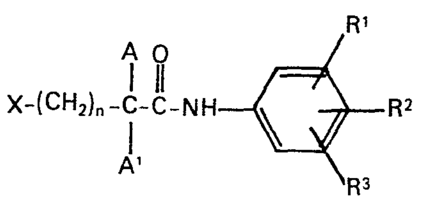

I

dans laquelle
X représente un radical éthényle ou éthynyle, un ou deux atomes d'hydrogène du radical éthényle ou un atome d'hydrogène du radical éthynyle pouvant être remplacés par un méthyle et/ou un éthyle,
A et $A^1$, identiques ou différents, sont choisis parmi l'hydrogène et les radicaux méthyle et éthyle,
n = 1
$R^1$ est un atome d'hydrogène ou d'halogène, un radical alkyle à chaîne droite ou ramifiée en $C_{1-4}$, éventuellement halogéné, un alcoxy en $C_{1-4}$, un aryloxy éventuellement halogéné ou un groupe nitro, et
$R^2$ et $R^3$, qui peuvent être identiques ou différents l'un de l'autre, sont choisis parmi l'hydrogène, les halogènes et des radicaux alkyles et alcoxy en $C_{1-4}$.

6. Variante de l'utilisation selon l'une quelconque des revendications 1 à 5, caractérisée en ce que dans le radical anilino des acylanilides:
$R^1$ représente H, F, Cl ou Br, un méthyle, éthyle, propyle ou isopropyle, un trifluorométhyle, un méthoxy, un phénoxy éventuellement chloré ou un groupe nitro, et
$R^2$ et $R^3$, identiques ou différents, sont choisis parmi l'hydrogène, le chlore et les radicaux méthyle et éthyle.

7. Produits phytosanitaires contenant le 3',4'-dichloroanilide du 2,2-diméthylpenténoïque-3.

8. Les acylanilides de formule générale

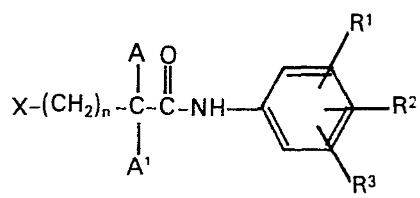

I

dans laquelle
X désigne un radical $H-C{\equiv}C-$ dont l'atome d'hydrogène peut être remplacé par un groupe méthyle ou éthyle,
A et $A^1$, qui peuvent être identiques ou différents l'un de l'autre, sont choisis parmi l'hydrogène et les groupes méthyle et éthyle,
n = 0 ou 1,
$R^1$ représente un atome d'hydrogène ou d'halogène, un alkyle droit ou ramifié en $C_{1-4}$, éventuellement halogéné, un alcoxy en $C_{1-4}$, un aryloxy éventuellement halogéné ou un groupe nitro, et
$R^2$ et $R^3$, identiques ou différents, sont choisis parmi l'hydrogène, les halogènes et des alkyles et alcoxy en $C_{1-4}$.

9. Produits phytosanitaires caractérisés en ce qu'ils contiennent un ou plusieurs acylanilides selon la revendication 8.

10. Procédé de préparation des composés selon la revendication 8, caractérisé en ce que l'on transforme les acides carboxyliques libres

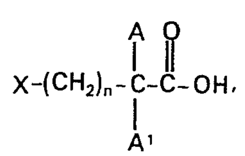

formule dans laquelle X, A, $A^1$ et n ont les significations précitées,
en chlorure d'acides avec lesquels on forme les anilides correspondants, éventuellement en présence d'un agent liant les acides.

**Revendications** pour l'Etat contractant: AT

1. Utilisation comme agents phytosanitaires d'un ou de plusieurs acylanilides de formule générale

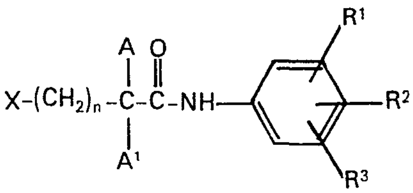
I

dans laquelle
X représente un radical éthényle ou éthynyle, un ou deux atomes d'hydrogène du radical éthényle ou un atome d'hydrogène du radical éthynyle pouvant être remplacés par un méthyle et/ou un éthyle;
A et $A^1$, qui peuvent être identiques ou différents l'un de l'autre, représentent chacun l'hydrogène ou le radical méthyle ou éthyle;
n est le nombre 0 ou 1;
$R^1$ désigne un atome d'hydrogène ou d'halogène, un alkyle en $C_{1-4}$ à chaîne droite ou ramifiée, éventuellement halogéné, un alcoxy en $C_{1-4}$, un radical aryloxy éventuellement halogéné ou un groupe nitro; et
$R^2$ et $R^3$, qui peuvent être identiques ou différents l'un de l'autre, représentent chacun l'hydrogène ou un halogène ou un alkyle ou un alcoxy en $C_{1-4}$.

2. Variante de la revendication 1, caractérisée en ce que l'on utilise des acylanilides dans le radical acyle desquels, de formule générale

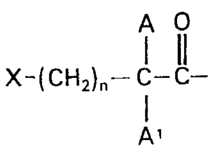
II

X est un radical $Y_2C{=}CH-$ à restes Y identiques ou différents, à savoir H, $CH_3$ ou $C_2H_5$,
n est le nombre 0 ou 1
et
A et $A^1$ représentent chacun l'hydrogène ou un méthyle.

3. Variante de la revendication 2, caractérisée en ce que le nombre des atomes de carbone de l'ensemble des deux restes Y est égal ou inférieur 2.

4. Utilisation comme herbicides d'un ou de plusieurs acylanilides de formule générale

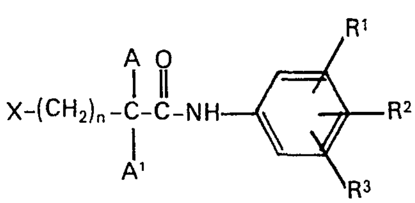
I

dans laquelle
X représente un radical éthényle ou éthynyle, un ou deux atomes d'hydrogène du radical éthényle ou un atome d'hydrogène du radical éthynyle pouvant être remplacés par un méthyle et/ou un éthyle,
A ou $A^1$, mais de préférence les deux, représentent chacun un méthyle,
n = 0,
$R^1$ est un atome d'hydrogène ou d'halogène, un radical alkyle à chaîne droite ou ramifiée en $C_{1-4}$, éventuellement halogéné, un alcoxy en $C_{1-4}$, un aryloxy éventuellement halogéné ou un groupe nitro, et
$R^2$ et $R^3$, qui peuvent être identiques ou différents l'un de l'autre, sont choisis parmi l'hydrogène, les halogènes et des radicaux alkyles et alcoxy en $C_{1-4}$.

5. Utilisation comme fongicides d'un ou de plusieurs acylanilides de formule générale

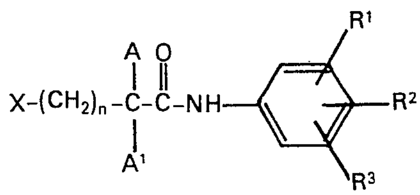
I

dans laquelle
X représente un radical éthényle ou éthynyle, un ou deux atomes d'hydrogène du radical éthényle ou un atome d'hydrogène du radical éthynyle pouvant être remplacés par un méthyle et/ou un éthyle,
A et $A^1$, identiques ou différents, sont choisis parmi l'hydrogène et les radicaux méthyle et éthyle,
n = 1
$R^1$ est un atome d'hydrogène ou d'halogène, un radical alkyle à chaîne droite ou ramifiée en $C_{1-4}$, éventuellement halogéné, un alcoxy en $C_{1-4}$, un aryloxy éventuellement halogéné ou un groupe nitro, et
$R^2$ et $R^3$, qui peuvent être identiques ou différents l'un de l'autre, sont choisis parmi l'hydrogène, les halogènes et des radicaux alkyles et alcoxy en $C_{1-4}$.

6. Variante de l'utilisation selon l'une quelconque des revendications 1 à 5, caractérisée en ce que dans le radical anilino des acylanilides:
$R^1$ représente H, F, Cl ou Br, un méthyle, éthyle, propyle ou isopropyle, un trifluorométhyle, un méthoxy, un phénoxy éventuellement chloré ou un groupe nitro, et
$R^2$ et $R^3$, identiques ou différents, sont choisis parmi l'hydrogène, le chlore et les radicaux méthyle et éthyle.

7. Produits phytosanitaires contenant le 3',4'-dichloroanilide du 2,2-diméthylpenténoïque-3.

8. Procécé de préparation d'acylanilides de formule générale

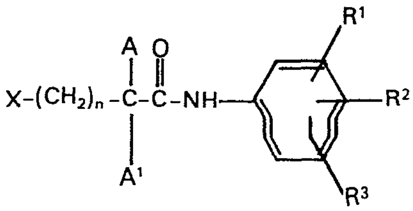

I

dans laquelle
X désigne un radical H-C≡C- dont l'atome d'hydrogène peut être remplacé par un groupe méthyle ou éthyle,
A et $A^1$, qui peuvent être identiques ou différents l'un de l'autre, sont choisis parmi l'hydrogène et les groupes méthyle et éthyle,
n = 0 ou 1,
$R^1$ représente un atome d'hydrogène ou d'halogène, un alkyle droit ou ramifié en $C_{1-4}$, éventuellement halogéné, un alcoxy en $C_{1-4}$, un aryloxy éventuellement halogéné ou un groupe nitro, et
$R^2$ et $R^3$, identiques ou différents, sont choisis parmi l'hydrogène, les halogènes et des alkyles et alcoxy en $C_{1-4}$,
par conversion des acides caboxyliques libres

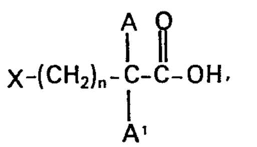

formule dans laquelle X, A, $A^1$ et n ont les significations précitées,
en leurs chlorures d'acides, avec lesquels on forme ensuite les anilides correspondants, éventuellement en présence d'un agent liant les acides.

9. Produits phytosanitaires caractérisés en ce qu'ils contiennent un ou plusieurs acylanilides de formule générale

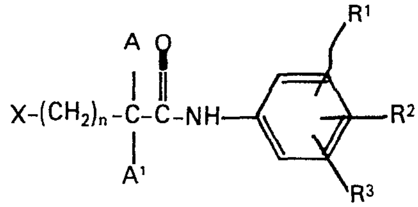

I

dans laquelle
X désigne un radical H-C≡C- dont l'atome d'hydrogène peut être remplacé par un groupe méthyle ou éthyle,
A et $A^1$, qui peuvent être identiques ou différents l'un de l'autre, sont choisis parmi l'hydrogène et les groupes méthyle et éthyle,
n = 0 ou 1,
$R^1$ représente un atome d'hydrogène ou d'halogène, un alkyle droit ou ramifié en $C_{1-4}$, éventuellement halogéné, un alcoxy en $C_{1-4}$, un aryloxy éventuellement halogéné ou un groupe nitro, et
$R^2$ et $R^3$, identiques ou différents, sont choisis parmi l'hydrogène, les halogènes et des alkyles et alcoxy en $C_{1-4}$.

10. Produits phytosanitaires selon la revendication 9, caractérisés en ce que dans la formule I:
X désigne un radical H-C≡C-,
n = 0 ou 1,
A et $A^1$, identiques ou différents, sont chacun l'hydrogène ou un méthyle,
$R^1$ représente H, F, Cl ou Br, un méthyle, éthyle, propyle ou isopropyle, un trifluorométhyle, un méthoxy, un phénoxy éventuellement chloré ou un groupe nitro, et
$R^2$ et $R^3$, identiques ou différents, sont choisis parmi l'hydrogène, le chlore et les radicaux méthyle et éthyle.